# EUROPEAN PATENT APPLICATION

(11) **EP 4 809 941 A1**
(43) Date of publication of application: **23.09.2026**
(21) Application number: 26165003.0
(22) Date of filing: 16.03.2026
(51) Int. Cl.: A61B 17/28, A61B 90/57

(54) **CLAMP ASSEMBLY**

(30) Priority: 16.03.2025 US 202563772595 P
(71) Applicant: LSI Solutions, Inc., Victor, NY 14564 (US)
(72) Inventor: SAUER, Jude S., Pittsford, 14534 (US)
(74) Representative: Flügel Preissner Schober Seidel

(57) **Abstract**

A clamp assembly (10, 100) includes an elongated body portion (21), a primary clamp jaw (16a) coupled to the body portion (21), and a secondary clamp jaw (16b, 104) that includes a base portion (106) having a portion pivotably coupled to the body portion (21) such that the secondary clamp jaw (16b, 104) is pivotable between a first position and a second position. The secondary clamp jaw (16b, 104) includes a first flexible member (122a) and a second flexible member (122b), with a proximal end of the first and second flexible members (122a, 122b) coupled to corresponding portions of the base portion (106). The secondary clamp jaw (16b, 104) also includes an elongated first clamp arm (112a) and second clamp arm (112b) that each extends from a distal end of the first and second flexible members (122a, 122b), respectively, such that a distal end of the first and second clamp arms (112a, 112b) is configured to displace relative to the corresponding portions of the base portion (106).

## Description

### REFERENCE TO RELATED APPLICATIONS

This patent application is a continuation-in-part of U.S. Patent Application No. 18/949,012, filed November 15, 2024, which is a continuation of U.S. Patent Application No. 17/635,461, filed February 15, 2022, which is a national stage application, filed under 35 U.S.C. § 371, of International Patent Application No. PCT/US20/22700, filed on March 13, 2020, which claims priority to U.S. Provisional Patent Application No. 62/817,967 filed March 13, 2019, and U.S. Provisional Patent Application No. 62/827,352 filed April 1, 2019, the contents of each of which is incorporated by reference herein in its entirety. This application also claims priority to U.S. Provisional Patent Application No. 63/772,595, filed March 16, 2025 and entitled "CLAMP ASSEMBLY", the contents of which is hereby incorporated by reference in its entirety.

### FIELD

The claimed invention relates to surgical devices, and more specifically to a surgical access device or system useful in the correction of tricuspid regurgitation and methods thereof.

### BACKGROUND

Clamps assemblies are frequently used during surgical procedures to secure objects that are used in a given procedure. Typically, clamp assemblies include a first clamp arm and a second clamp arm, and an object is positioned between a contact surface of the first clamp arm and a contact surface of the second clamp arm with the first and second clamp arms in an open position. When the first and second clamp arms are pivoted into a closed position, the contact surface of the first clamp arm contacts a first portion of the surface of the object and the contact surface of the second clamp arm contacts a second portion of the surface of the object to secure the object.

When the object has a regularly shaped configuration, the first portion of the surface of the object and the second portion of the surface of the object may be easily aligned with the contact surface of the first clamp arm the contact surface of the second clamp arm, respectfully, to secure the object. However, when the object has an irregularly shaped configuration, the contact surface of the first clamp arm may not suitably align with the first portion of the surface of the object and/or the contact surface of the second clamp arm may not suitably align with the second portion of the surface of the object, and as a result, the object may not be sufficiently clamped by the clamp assembly. These insufficiently-secured objects result in wasted time as personnel repeatedly attempt to secure the object using the conventional clamp assembly. Further, when the object is not fully secured by the clamp assembly, the object may move during the procedure, which may result in the need to resecure or reposition the object multiple times during a procedure. In some instances, such insufficiently-secured objects may be completely dislodged, which may cause damage to the object, which may be salivate and expensive, or may cause injury to a patient or a surgeon. Accordingly, there is a need for a clamp assembly that allows irregularly shaped objects to be quickly and easily securely grasped during a procedure.

### SUMMARY OF THE INVENTION

The invention is set out in the appended claims.

### BRIEF DESCRIPTION OF THE DRAWINGS

FIG. 1A is an exploded view illustrating an assembly step for a surgical access system using an embodiment of a clamp assembly;
FIG. 1B is a perspective view of the surgical access system using the claim assembly of FIG. 1A;
FIGS. 2A to 2F are various views of a further embodiment of a clamp assembly;

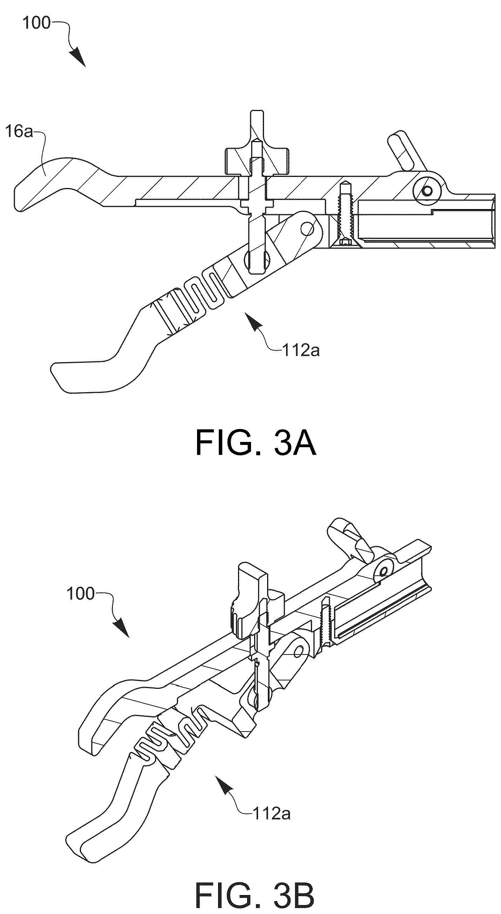

FIG. 3A is a sectional view of the clamp assembly taken along section line 3A-3A of FIG. 2B;
FIG. 3B is a perspective view of the sectional view of the clamp assembly of FIG 3A;
FIG. 4A is a front view of a secondary clamp jaw of the embodiment of the clamp assembly of FIG. 2A;
FIG. 4B is a side view of the secondary clamp jaw;
FIG. 4C is a perspective view of the secondary clamp jaw; and
FIG. 5 is view of the embodiment of the clamp assembly of FIG. 2A engaging an irregularly shaped object.

### DETAILED DESCRIPTION

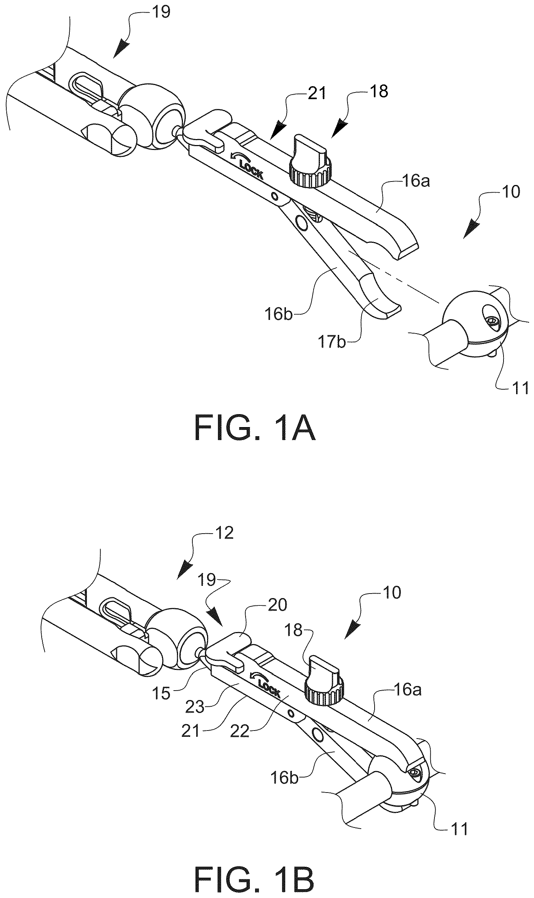

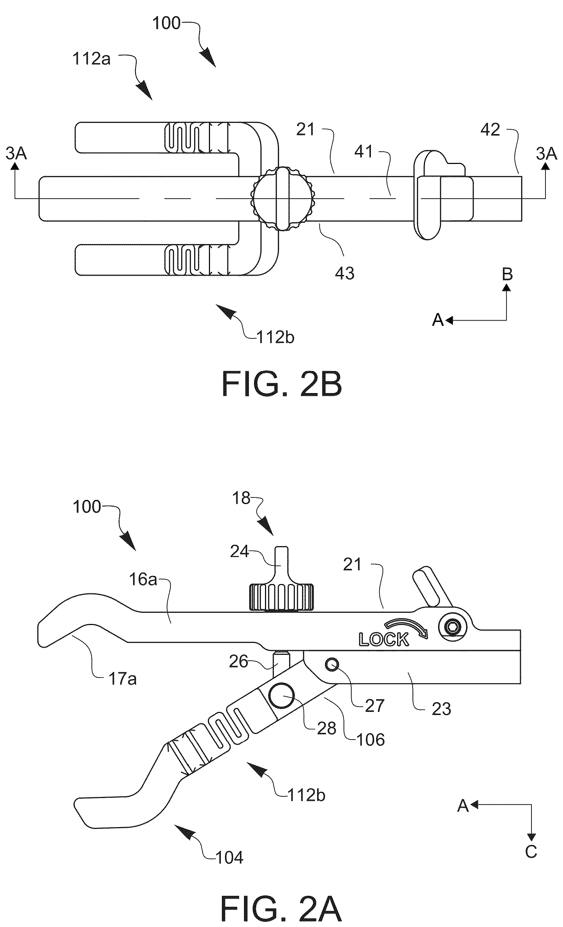

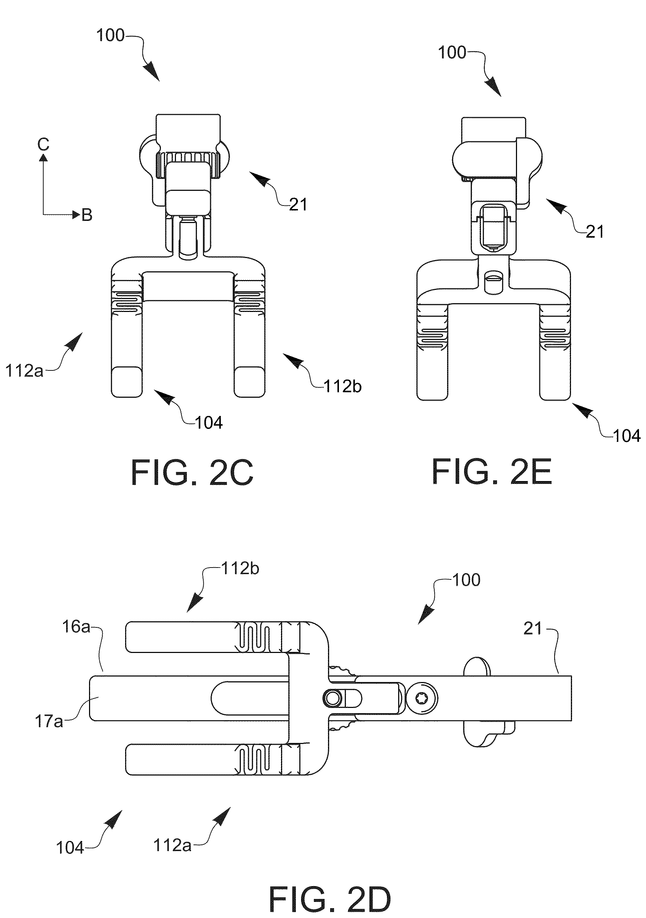

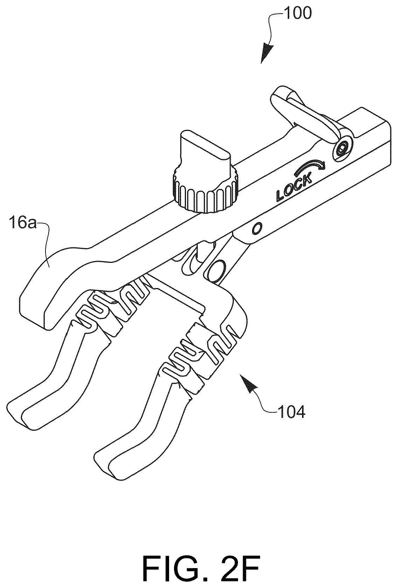

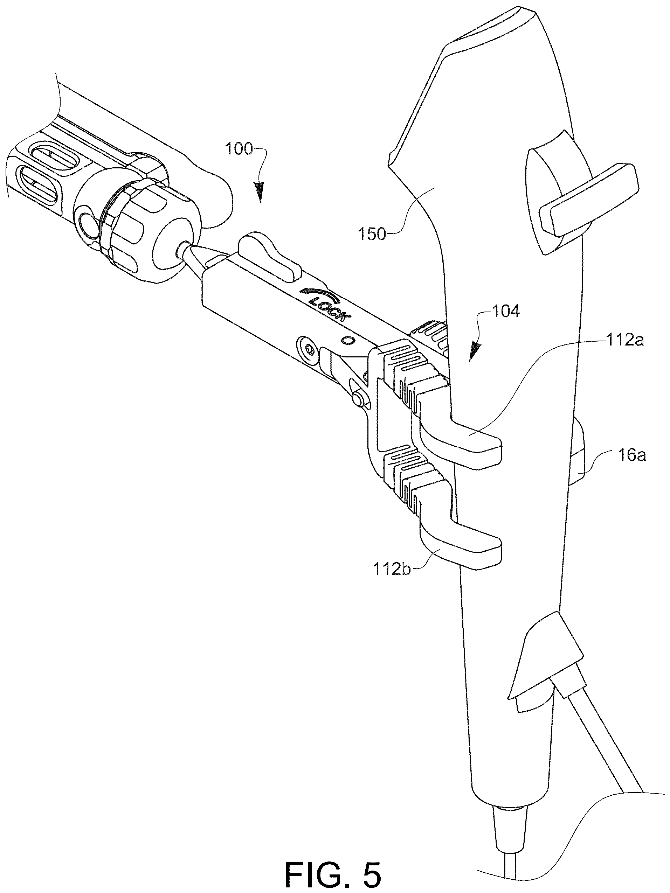

FIGS. 2A to 2F illustrate an embodiment of a clamp assembly 100 that may be removably secured to a medical device, such as a surgical instrument holder 12 (see FIG 1B). The clamp assembly 100 includes an elongated body portion 21 that extends from a distal end to a proximal end. A primary clamp jaw 16a extends from a distal end to a proximal end, with the proximal end of the primary clamp jaw 16a being coupled to a first portion of the body portion 21 at or adjacent to the distal end of the body portion 21, and with a portion of the primary clamp jaw forming a primary contact surface 17a (see Fig 2A). The clamp assembly 100 further includes a secondary clamp jaw 104 comprising a base portion 106, wherein a portion of the base portion 106 is pivotably coupled to a second portion of the body portion 21 such that the secondary clamp jaw is pivotable between a first position (see Fig. 2A) and a second position (see Fig 5).

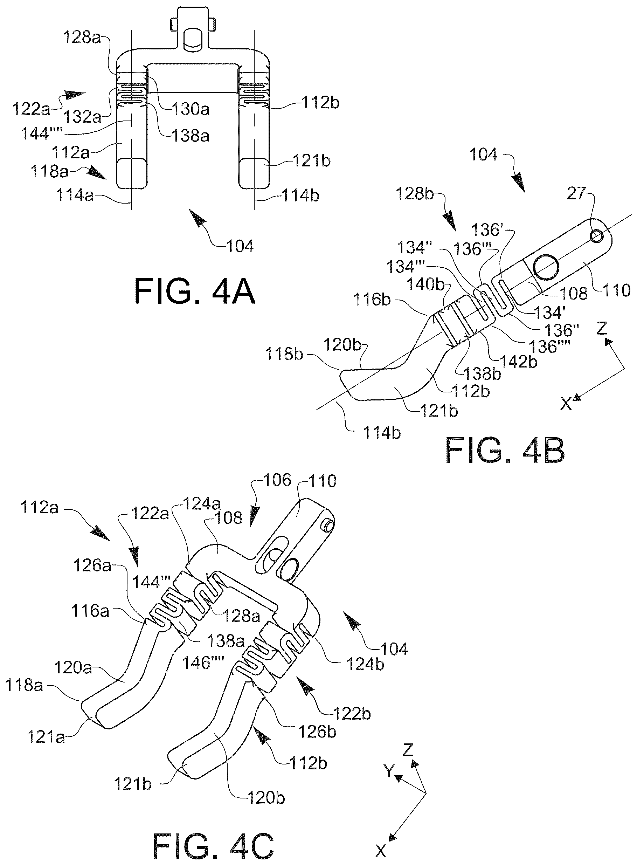

With reference to FIG. 4C, the secondary clamp jaw 104 includes a first flexible member 122a, wherein a proximal end 124a of the first flexible member 122a is coupled to a first portion of the base portion 106, and an elongated first clamp arm 112a that extends along a first clamp arm axis 114a (see FIG. 4A) from a proximal end 116a to a distal end 118a, with the proximal end 116a of the first clamp arm 112a being coupled to a distal end 126a of the first flexible member 122a such that the distal end 118a of the first clamp arm 112a is configured to displace relative to the first portion of the base portion 106, wherein a first engagement portion 121a extends along a portion of the first clamp arm 112a, with a first arm contact surface 120a extending along a portion of the first engagement portion 121a, the first arm contact surface 120a being an upwardly-facing surface of the first engagement portion 121a.

Referring again to FIG. 4C, the secondary clamp jaw 104 further includes a second flexible member 122b, wherein a proximal end 124b of the second flexible member 122b is coupled to a second portion of the base portion 106, and an elongated second clamp arm 112b that extends along a second clamp arm axis 114b (see FIG. 4A) from a proximal end 116b to a distal end 118b (see FIG. 4B), with the proximal end 116b of the second clamp arm 112b being coupled to a distal end 126b of the second flexible member 122b such that the distal end 118a of the second clamp arm 112b is configured to displace relative to the second portion of the base portion 106, wherein a second engagement portion 121b extends along a portion of the second clamp arm 112b, with a second arm contact surface 120b extending along a portion of the second engagement portion 121b, the second arm contact surface 120b being an upwardly-facing surface of the second engagement portion 121b. When the secondary clamp jaw 104 is pivoted from the first position to the second position, a portion of the primary contact surface 17a is configured to contact a first portion of an object, such as the irregularly-shaped surgical device 150 of Fig. 5, a portion of the first arm contact surface 120a is configured to contact a second portion of the object, and a portion of the second arm contact surface 120b is configured to contact a third portion of the object to secure the object.

So configured, the first and second clamp arms 112a, 112b are each capable of axial (e.g., along the X-axis of the reference coordinate system of FIGS. 4B and 4C) displacement (i.e., compression and extension) relative to the base portion 106, as well as bending an/or displacement of the distal ends 118a, 118b of the first and second clamp arms 112a, 112b normal to the X-axis of the reference coordinate system of FIGS. 4B and 4C, thereby providing vertical and axial fine adjustments to the shape and location of the contact surfaces 120a, 120b of the first and second clamp arms 112a, 112b, respectively. This design allows for a more universal holding system to accommodate different shapes and sizes of intended object to hold during surgery.

Turning first to the clamp assembly 10 illustrated in Figs. 1A and 1B, the clamp assembly 10 includes the elongated body portion 21 that may extend from a proximal end 42 to a distal end 43 along a body axis 41 (see Fig. 2B). The body portion 21 may include an elongated upper body portion 22 that extends from a distal end to a proximal end, and the proximal end of the upper body portion 22 may be at or adjacent to the proximal end 42 of the body portion 21 and the distal end of the upper body portion 22 may be at or adjacent to the distal end 43 of the body portion 21. A primary clamp jaw 16a may extend from a distal end to a proximal end along an axis that is aligned with or parallel to the body axis 41, with the proximal end of the primary clamp jaw 16a being integrally formed with a portion of the distal end of the upper body portion 22. The body portion 21 may also include a lower body portion 23 that extends from a distal end to a proximal end, and the lower body portion 23 is coupled to the upper body portion 22 such that the proximal end of the lower body portion 23 is aligned with the proximal end of the upper body portion 22, and the distal end of the lower body portion 23 is disposed at or adjacent to the distal end of the upper body portion 22.

With reference to FIG. 1B, a coupling portion 19 may be formed at or adjacent to a proximal portion of the body portion 21, and the coupling portion 19 may be configured to engage (or removably engage) a corresponding coupling portion 15 of a medical device. For example, the coupling portion 19 may be configured to receive a portion 15 of a surgical instrument holder 12, which may be configured to stably secure the clamp assembly 10 in a desired position. In some embodiments, the coupling portion 19 may be a receiving cavity (not shown) formed in the proximal end of the body portion 21 that is configured to receive a projection of the portion 15 of a surgical instrument holder 12, and the receiving cavity 19 may extend from the proximal end of the upper body portion 22 and the lower body portion 23 towards the distal end of the upper body portion 22 and the lower body portion 23. In some embodiments, a cam locking mechanism 20 may be selectively engaged to lock the end portion of the medical device within the receiving cavity 19. The surgical equipment holder 12 may be the miniARM^{™} INSTRUMENT HOLDER from LSI Solutions, Inc. (Victor, NY, www.lsisolutions.com) that is intended for use in such surgical procedures.

The clamp assembly 10 may also include a secondary clamp jaw 16b that extends from a distal end to a proximal end, and the proximal end of the secondary clamp jaw 16b is pivotably coupled to the distal end of the lower body portion 23. The pivoting of the secondary clamp jaw 16b relative to the primary clamp jaw 16a may be controlled in any suitable manner. In some embodiments, an adjustment knob assembly 18 may be coupled to a portion of the primary clamp jaw 16a and the secondary clamp jaw 16b to pivot the secondary clamp jaw 16b away from or towards the primary clamp jaw 16a.

With reference to FIG. 2A, which illustrates a further embodiment of the clamp assembly 100 which will be discussed in more detail below, the adjustment knob assembly 18 may include a knob portion 24 that may be fixedly secured to an elongated threaded member 26 that may extend along a member axis, and the threaded member 26 may extend through an aperture formed through the primary clamp jaw 16a (or through the upper body portion 22). As the knob portion 24 is rotated in a first rotational direction, the threaded member 26 rotates about the member axis in the first rotational direction, and as the knob portion 24 is rotated in a second rotational direction, the threaded member 26 rotates about the member axis in the second rotational direction. A portion of the threaded member 26 may be received in, and threadedly engage, a threaded aperture (not shown) that is formed in a portion of the secondary clamp jaw 16b (or formed in a separate component, such as cylindrical pin 28 that is coupled to the secondary clamp jaw 16b) such that the portion of the secondary clamp jaw 16b (or the cylindrical pin 28) is linearly displaced along the threaded member 26 in a first linear direction when the threaded member 26 rotates about the member axis in the first rotational direction and is linearly displaced along the threaded member 26 in a second linear direction when the threaded member 26 rotates about the member axis in the second rotational direction.

So configured, the adjustment knob assembly 18 may be rotated in the first direction to pivot the distal end of the secondary clamp jaw 16b away from the distal end of the primary clamp jaw 16a to move the clamp assembly into a first, or an open, position, as illustrated in FIG. 1A. In this open position, a base member, such as a ball joint 11, may be positioned between a contact surface 17a (see FIG. 2A) of the primary clamp jaw 16a and a contact surface 17b of the secondary clamp jaw 16b. When the clamp assembly 10 is moved into a suitable position relative to the base member, the adjustment knob assembly 18 may be rotated in the second direction to pivot the distal end of the secondary clamp jaw 16b towards the distal end of the primary clamp jaw 16a. When a first portion of the base member contacts the contact surface 17a of the primary clamp jaw 16a and a second portion of the base member contacts the contact surface of the secondary clamp jaw 16b such that the base member is secured (and cannot move or displace relative to) the contact surface 17a of the primary clamp jaw 16a the contact surface of the secondary clamp jaw 16b, the clamp assembly 10 is in a second, or closed, position, illustrated in FIG. 1B.

In some embodiments, one or both of the contact surface 17a of the primary clamp jaw 16a and the contact surface 17b of the secondary clamp jaw 16b may each have a concave shape that may form a portion of a sphere having the same diameter as the ball joint 11, the clamp assembly 10 may be positioned in any desired orientation about the ball joint 11. Thus, the medical device, such as the surgical instrument holder 12, may be positioned in any desired orientation about the ball joint 11, which itself may be coupled to a second medical device. The clamp assembly 10 may be used in procedures described in, for example, U.S. Patent App. No. 16/610,352, filed on November 1, 2019, and U.S. Patent App. No. 17/635461, filed on February 15, 2022, and each of these applications is incorporated by reference in its entirety.

A further embodiment of a clamp assembly 100 is illustrated in FIG. 2A to 2F. The clamp assembly 100 may be identical or substantially identical to the clamp assembly 10 of FIGS. 1A and 1B, except that the clamp assembly 100 includes a secondary clamp jaw 104 that includes a first clamp arm 112a and a second clamp arm 112b that are coupled to a base portion 106 of the secondary clamp jaw 104 by a first flexible member 122a and a second flexible member 122b, respectively, thus allowing each of the first clamp arm 112a and the second clamp arm112b to move relative to the base portion 106 (and the primary clamp jaw 16a), thereby allowing the clamp assembly 100 to tightly grasp irregularly shaped objects, as illustrated in FIG. 5.)

Turning to the clamp assembly 100 in more detail, the secondary clamp jaw 104 is illustrated in FIGS. 4A to 4C. The secondary clamp jaw 104 includes the base portion 106 that includes a cross member 108 that extends along the Y-axis of the reference coordinate system of FIG. 4C from a first end to a second end. A support member 110 may extend from (e.g., normal to) the cross member 108, such as a point midway between the first end and the second end of the cross member 108. In some embodiments, the support member 110 may extend along or parallel (or substantially along or substantially parallel) to the X-axis of the reference coordinate system of FIGS. 4B and 4C. Referring to FIG. 2A, a pin 27 may extend through a proximal end of the support member 110, and the pin 27 may extend along a pivot axis that may be parallel to the Y-axis of the reference coordinate system of FIG. 4C (and the B-axis of the reference coordinate system of FIG. 2B and 2C). The pin 27 may be coupled to a portion of the lower body portion 23 to pivotably couple the secondary clamp jaw 104 to the lower body portion 23 such that the secondary clamp jaw 104 pivots about the pivot axis.

As illustrated in FIG. 4C, which illustrates the secondary clamp jaw 104, the first clamp arm 112a may extend from a first portion of the cross member 108, such as the first end of the cross member 108, and a second clamp arm 112b may extend from a second portion of the cross member 108, such as the second end of the cross member 108. Each of the first clamp arm 112a and the second clamp arm 112b may be identical, and therefore only the first clamp arm 112a is described in detail, but it is understood that the second clamp arm has identical features to the first clamp arm 112a. In addition, features of the first clamp arm 112a will have an "a" following a particular reference number, and features of the second clamp arm 112b will have a "b" following that particular reference number.

The first clamp arm 112a may extend along or generally along a first clamp arm axis 114a (see FIG. 4A) from a proximal end 116a to a distal end 118a, and the first clamp arm axis 114a may extend along the X-axis of the reference coordinate system of FIGS. 4B and 4C. An engagement portion 121a may extend along a portion of the first clamp arm 112a, and the engagement portion 121a may extend from the distal end 118a of the first clamp arm 112a to the proximal end 116a of the of the first clamp arm 112a or to an intermediate point of the first clamp arm 112a that is disposed between the proximal end 116a and the distal end 118a of the of the first clamp arm 112a. A contact surface 120a may extend along a portion of the engagement portion 121a, such as an upwardly-facing surface of the engagement portion 121a. In some embodiments the contact surface 120a may extend from a distal end to a proximal end, and the distal end may be at or adjacent to the distal end 118a of the first clamp arm 112a and/or the distal end of the engagement portion 121a. The proximal end may be at or adjacent to the proximal end of the engagement portion 121a, or may be distal to the proximal end of the engagement portion 121a. In some embodiments, the contact surface 120a may have a substantial U-shape, V-shape, rounded, or a semi-circular shape when viewed along the Y-axis of the reference coordinate system of FIG. 4C, such as the view of FIG. 4B. That is, the contact surface 120a may have a contoured shape having a substantial U-shape or V-shape, semi-circular, or rounded shape when viewed in a cross-section taken along the X-Z plane of the reference coordinate system of FIG. 4C. In some embodiments, the contact surface 120a may have a contoured shape having a substantial U-shape or V-shape, semi-circular, or rounded shape when viewed in a cross-section that is normal to first clamp arm axis 114a (e.g., a cross-section taken along the Y-Z plane of the reference coordinate system of FIGS. 4B and 4C). However, the contact surface 120a may have any suitable shape or combination of shapes that are suitable to engage a surface, such as a contoured or an irregular surface. The contact surface 120a may have features that assist in gripping, and may have a layer of material applied to the surface of the contact surface 120a to assist in gripping.

The first clamp arm 112a may be secured to a first flexible member 122a disposed between a portion of the first clamp arm 112a and/or a portion of the cross member 108. The first flexible member 122a may extend from a proximal end 124a to a distal end 126a, and the distal end 126a of the first flexible member 122a may be disposed at the proximal end 116a of the first clamp arm 112a, or at the proximal end of the engagement portion 121a such that the first flexible member 122a is disposed between the engagement portion 121a and the first end of the cross member 108. The first flexible member 122a may allow the first clamp arm 112a to displace, flex, bend, articulate, and/or pivot relative to the cross member 108 and/or to the to the proximal end 124a of the first flexible member 122a, thereby allowing the contact surface 120a to independently displace (relative to the primary clamp jaw 16a) to more securely engage or grasp a surface of an object.

The first flexible member 122a may be any shape, material, or combination of shapes and materials that will allow the first clamp arm 112a to displace, flex, bend, articulate, and/or pivot relative to the proximal end 124a of the first flexible member 122a and/or a portion of the cross member 108. In some embodiments, the first flexible member 122a may be a spring, such as a leaf spring or a coil spring, for example. In some embodiment, the spring may be integrally formed with the first clamp arm 112a and the cross-member 108 such that the secondary clamp jaw 104 is a single, unitary part. In other examples, the spring may be coupled to one or more portions of the first clamp arm 112a and/or the cross member 108. In other examples, the first flexible member 122a may include a flexible material that is different than the material of the first clamp arm 112a and/or the material of the cross-member 108.

With reference to FIG. 4B, the first flexible member 122a may include a first spring portion 128a, which may have a cross-section (taken along the X-Z-plane of the reference coordinate system of FIG. 4C) that may be constant or substantially constant from an inner lateral end 130a (see FIG. 4A) to an outer lateral end 132a (i.e., along the Y-axis of the reference coordinate system of FIG. 4C). This cross-section, when viewed along the along the Y-axis of the reference coordinate system of FIG. 4B which shows the second flexible member 122b, includes two or more parallel linear segments 134, and a single end of each linear segment 134 may be coupled to an adjacent end of an adjacent linear segment 134. For example, a first end of a first linear segment 134' may be coupled to a portion of the cross member 108 by a first coupling segment 136' that extends from the first end of the first linear segment 134' to the portion of the cross member 108. The first coupling segment 136' may have any suitable shape or combination of shapes, and may be linear, or may be rounded or arched, or partially rounded or arched.

A second end of the first linear segment 134' may be coupled to a second end of a second linear segment 134" by a second coupling segment 136", which may have any suitable shape or combination of shapes, and may be linear, or may be rounded or arched, or partially rounded or arched. A first end of the second linear segment 134" may be coupled to a first end of a third linear segment 134‴ by a third coupling segment 136"', which may have any suitable shape or combination of shapes, and may be linear, or may be rounded or arched, or partially rounded or arched. In some embodiments having only a first spring portion 128a (not shown), the second end of the third linear segment 134‴ may be coupled to any point (or portion) of the proximal end 116a of the first clamp arm 112a by a fourth coupling segment 136"". More linear segments 134 (e.g., four linear segments, five linear segments, six linear segments, seven linear segment, etc.) or fewer linear segments 134 (e.g., two linear segments, one linear segment) may be included in the first flexible member 122a.

With reference again to FIG. 4C, the first flexible member 122a may also include a second spring portion 138a that may be distal to the first spring portion 128a, and the second spring portion 138a may have a proximal end that is coupled to a distal end of the first spring portion 128a and a distal end that is coupled to the proximal end 116a of the first clamp arm 112a. For example, the second end of the third linear segment 134‴ of the first spring portion 128a may be coupled to a portion of the second spring portion 138a at the proximal end of the second spring portion 138a by the fourth coupling segment 136"". So configured, the proximal end 124a of the first flexible member 122a may be the proximal end of the first spring portion 128a and the distal end 126a of the first flexible member 122a may be the distal end of the second spring portion 138a. The second spring portion 138a may be any spring or material described above, or may be identical (or substantially identical) to the first spring portion 128a with the exception that the second spring portion 138a may be rotated or angularly offset from the first spring portion 128a. For example, the orientation of the second spring portion 138a may be rotated 90 degrees from the orientation of the first spring portion 128a. That is, the second spring portion 138a may have a cross-section (taken along the X-Y plane of the reference coordinate system of FIGS. 4B and 4C) that may be constant or substantially constant from an upper lateral end 140a (see FIG 4B) to a lower lateral end 142a (i.e., along the Z-axis of the reference coordinate system of FIG. 4B). This cross-section, when viewed along the along the Z-axis of the reference coordinate system of FIG. 4B, includes two or more parallel linear segments 144, and the linear segments 144 and coupling segments 148 of the second spring portion 138a may be similar to or identical to those of the first spring portion 128a. However, the second spring portion 138a may have more or fewer linear segments 144 than the first spring portion 128a, or may have differently shaped coupling members 136 that the first spring portion 128a. In some embodiments, an end of the third linear segment 144'" of the second spring portion 138a may be coupled to a coupling segment 146 of the second spring portion 138a that may be coupled to the proximal end 116a of the first clamp arm 112a.

As previously discussed, the second clamp arm 112b may be identical or substantially identical to the first clamp arm 112a, and the second clamp arm 112b may have a second flexible member 122b that may be identical the first flexible member 122a.

However, the second flexible member 122b may not be identical to the first flexible member 122a and may include any or all of the materials or springs described with respect to the first flexible member 122a. In addition, the secondary clamp jaw 104 may be a single, unitary part that may be formed from any suitable material, such as injection molded plastic or a metal material, such as stainless steel.

So configured, and as previously explained, the first and second clamp arms 112a, 112b are each capable of axial displacement (i.e., compression and extension) relative to the base portion 106, as well as bending an/or displacement of the distal ends of the first and second clamp arms 112a, 112b normal to the X-axis of the reference coordinate system of FIGS. 4B and 4C, thereby providing vertical and axial fine adjustments to the shape and location of the contact surfaces 120a, 120b of the first and second clamp arms 112a, 112b, respectively.

Embodiments discussed herein have been described by way of example in this specification. It will be apparent to those skilled in the art that the forgoing detailed disclosure is intended to be presented by way of example only, and is not limiting. Various alterations, improvements, and modifications will occur and are intended to those skilled in the art, though not expressly stated herein. Additionally, the recited order of processing elements or sequences, or the use of numbers, letters, or other designations therefore, is not intended to limit the claims to any order, except as may be specified in the claims. Accordingly, the invention is limited only by the following claims and equivalents thereto.

## Claims

1. A clamp assembly (10, 100) comprising:
an elongated body portion (21) that extends from a distal end to a proximal end;
a primary clamp jaw (16a) that extends from a distal end to a proximal end , with the proximal end of the primary clamp jaw (16a) being coupled to a first portion of the body portion (21) at or adjacent to the distal end of the body portion (21) and with a portion of the primary clamp jaw (16a) forming a primary contact surface (17a); and
a secondary clamp jaw (16b, 104) comprising:
a base portion (106), wherein a first portion of the base portion (106) is pivotably coupled to a second portion of the body portion (21) such that the secondary clamp jaw (16b, 104) is pivotable between a first position and a second position;
a first flexible member (122a), wherein a proximal end of the first flexible member (122a) is coupled to a second portion of the base portion (106);
an elongated first clamp arm (112a) that extends along a first clamp arm axis (114a) from a proximal end to a distal end , with the proximal end of the first clamp arm (112a) being coupled to a distal end of the first flexible member (122a) such that the distal end of the first clamp arm (112a) is configured to displace relative to the second portion of the base portion (106), wherein a first engagement portion (121a) extends along a portion of the first clamp arm (112a), with a first arm contact surface (120a) extending along a portion of the first engagement portion (121a), the first arm contact surface (120a) being an upwardly-facing surface of the first engagement portion (121a);
a second flexible member (122b), wherein a proximal portion of the second flexible member (122b) is coupled to a third portion of the base portion (106); and
an elongated second clamp arm (112b) that extends along a second clamp arm axis (114b) from a proximal end to a distal end, with a proximal end of the second clamp arm (112b) being coupled to a distal end of the second flexible member (122b) such that the distal end of the second clamp arm (112b) is configured to displace relative to the third portion of the base portion (106), wherein a second engagement portion (121b) extends along a portion of the second clamp arm (112b), with a second arm contact surface (120b) extending along a portion of the second engagement portion (121b), the second arm contact surface (120b) being an upwardly-facing surface of the second engagement portion (121b),
wherein when the secondary clamp jaw (16b, 104) is pivoted from the first position to the second position, a portion of the primary contact surface (17a) is configured to contact a first portion of an object, a portion of the first arm contact surface (120a) is configured to contact a second portion of the object, and a portion of the second arm contact surface (120b) is configured to contact a third portion of the object to secure the object.

2. The clamp assembly (10, 100) of claim 1, the body portion (21) further comprising an elongated upper body portion (22) that extends from a distal end to a proximal end, and wherein the proximal end of the primary clamp jaw (16a) is coupled to a portion of the distal end of the upper body portion (22).

3. The clamp assembly (10, 100) of claim 2, wherein the proximal end of the primary clamp jaw (16a) is integrally formed with the portion of the distal end of the upper body portion (22).

4. The clamp assembly (10, 100) of claim 2 or 3, wherein the distal end of the upper body portion (22) is at the distal end of the body portion (21), and the proximal end of the upper body portion (22) is at the proximal end of the body portion (21).

5. The clamp assembly (10, 100) of any one of claims 2 to 4, the body portion (21) further comprising a lower body portion (23) that extends from a distal end to a proximal end , the lower body portion (23) being coupled to the upper body portion (22) such that the proximal end of the lower body portion (23) is aligned with the proximal end of the upper body portion (22), and the distal end of the lower body portion (23) is disposed at or adjacent to the distal end of the upper body portion (22);
wherein preferably the first portion of the base portion (106) of the secondary clamp jaw (16b, 104) is pivotably coupled to a portion of the lower body portion (23);
wherein further preferably the portion of the lower body portion (23) is at or adjacent to the distal end of the lower body portion (23).

6. The clamp assembly (10, 100) of any one of claims 2 to 5, further comprising:
an adjustment knob assembly (18) including a knob portion (24) that is fixedly secured to an elongated threaded member (26), wherein a first portion of the threaded member (26) extends through an aperture formed in the primary clamp jaw (16a) (or a third portion of the body portion (21)) and a second portion of the threaded member (26) threadedly engages a portion of the secondary clamp jaw (16b, 104) such that rotating the knob portion (24) in a first rotational direction pivots the secondary clamp jaw (16b, 104) in a first direction relative to the primary clamp jaw (16a), and such that rotating the knob portion (24) in a second rotational direction pivots the secondary clamp jaw (16b, 104) in a second direction relative to the primary clamp jaw (16a).

7. The clamp assembly (10, 100) of claim 6, wherein the second portion of the threaded member (26) threadedly engages a threaded aperture formed in the portion of the secondary clamp jaw (16b, 104).

8. The clamp assembly (10, 100) of claim 6, wherein the primary contact surface (17a) has a concave shape that is configured to engage a corresponding first portion of a spherical ball joint (11).

9. The clamp assembly (10, 100) of any one of claims 1 to 8, wherein the base portion (106) of the secondary clamp jaw (16b, 104) includes a support member (110) that extends from a proximal end to a distal end , and a cross member (108) that extends from the distal end of the support member (110), wherein the second portion of the base portion (106) is a first portion of the cross member (108) and the third portion of the base portion (106) is a second portion of the cross member (108);
wherein preferably the first portion of the cross member (108) is at a first end of the cross member (108) and the second portion of the cross member (108) is a second end of the cross member (108).

10. The clamp assembly (10, 100) of any one of claims 1 to 9, wherein the proximal end of the primary clamp jaw (16a) is integrally formed with the first portion of the body portion (21).

11. The clamp assembly (10, 100) of any one of claims 1 to 9, wherein the first flexible member (122a) is integrally formed with the second portion of the base portion (106) and the proximal end of the first clamp arm (112a), and the second flexible member (122b) is integrally formed with the third portion of the base portion (106) and the proximal end of the second clamp arm (112b).

12. The clamp assembly (10, 100) of any one of claims 1 to 11, wherein the first flexible member (122a) includes a first member first spring portion (128a) having a cross-sectional shape that includes two or more parallel linear segments (134, 144), and a single end of each linear segment (134) is coupled to an adjacent end of an adjacent linear segment (134), wherein the cross-sectional shape of the first member first spring portion (128a) is constant along a first axis; and
wherein the second flexible member (122b) includes a second member first spring portion (128b) having a cross-sectional shape that includes two or more parallel linear segments (134, 144), and a single end of each linear segment (134) is coupled to an adjacent end of an adjacent linear segment (134), wherein the cross-sectional shape of the second member first spring portion (128b) is constant along the first axis.

13. The clamp assembly (10, 100) of claim 12, wherein the first flexible member (122a) includes a first member second spring portion (138a) having a cross-sectional shape that includes two or more parallel linear segments (134, 144), and a single end of each linear segment (134) is coupled to an adjacent end of an adjacent linear segment (134), wherein the cross-sectional shape of the first member second spring portion (138a) is constant along a second axis that is normal to the first axis;
wherein the second flexible member (122b) includes a second member second spring portion (138b) having a cross-sectional shape that includes two or more parallel linear segments (134, 144), and a single end of each linear segment (134) is coupled to an adjacent end of an adjacent linear segment (134), wherein the cross-sectional shape of the second member second spring portion (138b) is constant along the second axis that is normal to the first axis.

14. The clamp assembly (10, 100) of any one of claims 1 to 13, wherein the first contact surface (120a) extends from a distal end to a proximal end , and the distal end is at or adjacent to the distal end of the first clamp arm (112a) and the proximal end is distally offset from the proximal end of the first clamp arm (112a); and
wherein the second contact surface (120b) extends from a distal end to a proximal end, and the distal end is at or adjacent to the distal end of the second clamp arm (112b) and the proximal end is distally offset from the proximal end of the second clamp arm (112b).

15. The clamp assembly (10, 100) of claim 14, wherein each of the first contact surface (120a) and the second contact surface (120b) has a substantial U-shape, a substantial V-shape, a substantial rounded shape, or a semi-circular shape when viewed normal to the first clamp arm axis (114a) or the second clamp arm axis (114b), respectively; or
wherein each of the first contact surface (120a) and the second contact surface (120b) has a contoured shape having a substantial U-shape, V-shape, semi-circular, or rounded shape when in cross-section normal to the first clamp arm axis (114a) or the second clamp arm axis (114b), respectively.
